# EUROPÄISCHE PATENTSCHRIFT

(11) **EP 1 872 760 B1**
(45) Veröffentlichungstag und Bekanntmachung des Hinweises auf die Patenterteilung: **16.05.2012**
(21) Anmeldenummer: 07010099.5
(22) Anmeldetag: 22.05.2007
(51) Int. Cl.: A61F 13/56

(54) **Verschlussband, Bandmaterial, Wicklung, Windel und Herstellungsverfahren**
Sealing strip, strip material, winding, napkin and manufacturing method
Bande de fermeture, matériau en bande, bobinage, couche et procédé de fabrication

(30) Priorität: 12.06.2006 DE 102006027452
(43) Veröffentlichungstag der Anmeldung: 02.01.2008
(73) Patentinhaber: Koester GmbH & Co. KG, 96146 Altendorf (DE)
(72) Erfinder: Magdalena, Drozdziok-Weinhardt, 91056 Erlangen (DE)
(74) Vertreter: Reuther, Martin

(56) Entgegenhaltungen:
- EP-A- 1 066 770
- WO-A-03/003962
- WO-A-2006/135281
- US-A1- 2003 100 878
- US-A1- 2005 283 954

## Beschreibung

Die Erfindung betrifft ein Verschlussband, ein Bandmaterial, eine Wicklung von Bandmaterial, eine Windel mit einem Verschlussband und ein Verfahren zum Herstellen eines Verschlussbandes.

Es hat sich bewährt, verschließbare Hygieneartikel mit Verschlussbändern auszustatten. Dies gilt insbesondere für Babywindeln und Inkontinenzwindeln, welche zum Anlegen bzw. zum Ablegen über die Hüfte des Trägers gezogen werden müssen und daher einen Verschließmechanismus erfordern. Mit einem Verschlussband, meist mit zwei Verschlussbändern, lässt sich dies leicht bewerkstelligen.

Ein hierzu verwendetes Verschlussband ist in der Regel in einem Befestigungsbereich permanent am Hygieneartikel befestigt, im Falle einer Windel üblicherweise an deren Windelohr. Hierzu dient meist eine Klebstoffverbindung zwischen dem Befestigungsbereich des Verschlussbandes und dem Hygieneartikel.

Die eigentliche Schließfunktion übernimmt ein Schließbereich am Verschlussband. Der Bereich ist definiert durch einen Bereich am freien Benutzerende des Verschlussbandes, welcher dazu eingerichtet ist, mit einer Oberfläche des Hygieneartikels lösbar verbunden zu werden, während der Befestigungsbereich permanent befestigt ist.

Moderne Windeln sind zum lösbaren Schließen mit einem mechanischen zwei-Komponenten-Verschlusssystem ausgerüstet, meist einem Haken-Schlaufen-Verschlusssystem, teilweise aber auch mit anderen ineinander verrastbaren Kunststoffkörpern, wie Pilzköpfen oder anderem. Während eine Komponente des mechanischen zwei-Komponenten-Verschlusssystems an der Windel angeordnet ist, hier in der Regel an einem Frontaltape, ist die am Verschlussband getragene Komponente auf einem Fasteningträger befestigt und hierzu in der Regel dort verklebt, wobei die US 2005/0283954 A1 ergänzend vorschlägt, die am Verschlussband getragene Komponente noch mittels eines Kompressionsvorganges mit dem Verschlussband zu verbinden. Bei einem solchen Verschlusssystem definiert sich der Schließbereich durch eine Umhüllende um die Hakenfläche.

Verschlussbänder, meist für Babywindeln oder Inkontinenzwindeln, zeigen unter anderem die DE 28 56 869 A1, die DE 197 32 499 A1, die DE 101 29 180 A1, die DE 101 40 621 A1, die DE 101 40 622 A1, die EP 0 233 704 A2, die EP 0 233 704 B1, die EP 0 235 014 B1, die EP 0 321 232 B1, die EP 0 418 951 B1, die EP 0 793 953 A2, die EP 0 795 307 A2, die EP 0 800 378 B1, die EP 0 840 585 B1, die EP 0 974 326 B1, die EP 1 000 598 A1, die EP 1 024 774 B1, die EP 1 484 041 A1, die EP 1 484 042 A1, die FR 2 586 558, die FR 2 606 257, die JP 7108-4 C, die US 6,210,389 B1, die US 5,624,429, die US 4,670,012, die US 4,959,265, die US 5,399,219, die US 5,611,790, die US 5,624,429, die US 5,676,652, die US 5,759,317, die US 5,997,522, die US 6,210,389 B1, die US 6,393,673 B1, die US 6,402,730 B 1, die US 6,428,525 B 1, die US 6,454,751 B 1, die US 2002/0095130 A1, die US 2003/0004490 A1, die US 2003/0009144 A1, die US 2003/0023322, die US 2003/0034583 A1, die US 2003/0060794, die WO 96/31181 A1, die WO 97/23186 A1, die WO 97/32555 A1, die WO 97/36566, die WO 99/06600, die WO 00/27330 A1, die WO 01/67911 A2, die WO 01/68019 A1, die WO 01/74283 A1, die WO 02/49567 A1, die WO 02/49568 A1 und die WO 03/003962 A1.

Um ein Verhaken der mechanischen Komponenten eines Verschlusssystems zu verbessern, indem diese flexibler ausgebildet werden, schlägt die US 2003/0100878 A1 unter anderem vor, unter der mechanischen Komponente kein Verschlussband vorzusehen und lediglich an den Rändern der mechanischen Komponente entsprechende Materialien anzubringen. Auch kann die mechanische Komponente, wie auch bei der EP 1 066 770 A1, gezielt geschwächt werden, um die Flexibilität zu erhöhen. Ähnliches offenbart auch die WO 03/003962 A1.

Einen anderen Ansatz wählt die WO 2006/135281 A1, welche in den Verschlussbändern Öffnungen vorschlägt, durch welche weitere Verschlussbänder hindurchgesteckt werden können, um auf diese Weise mittels mehrfacher mechanischer Verbindungen einen festen Sitz einer Windel sicherzustellen.

Der Erfindung liegt die Aufgabe zugrunde, ein verbessertes Verschlussband zur Verfügung zu stellen.

Die gestellte Aufgabe löst ein Verschlussband mit einer auf einem Fasteningträger getragenen Komponente eines mechanischen zwei-Komponenten-Verschlusssystems, wobei der Fasteningträger unter der Verschlusssystem-Komponente eine Ausstanzung aufweist.

Die entsprechenden Vorteile hat insbesondere in einer speziellen Ausführungsform der oben genannten Lösung ein Verschlussband für einen Hygieneartikel, insbesondere für eine Babywindel oder eine Inkontinenzwindel, mit einem Befestigungsbereich zum permanenten Befestigen am Hygieneartikel und mit einem Schließbereich zum gleichzeitigen lösbaren Verbinden mit einer Oberfläche des Hygieneartikels mit einer auf einem Fasteningträger getragenen Komponente eines mechanischen zwei-Komponenten-Verschlusssystems, wobei der Fasteningträger im Schließbereich ein Fenster aufweist und die Verschlusssystem-Komponente über dem Fenster kontinuierlich ist.

Begrifflich sei hierzu zunächst erläutert, dass unter der Ausnehmung eine makroskopische Veränderung des Trägers verstanden werden soll. Es wird davon ausgegangen, dass ein Fasteningträger, der in der Regel aus einer Kunststoffmischung hergestellt ist, in der Praxis häufig an der Bandoberfläche oder im Inneren mikroskopisch porenbehaftet sein wird. Eine makroskopische Ausnehmung ist hiervon jedoch zu unterscheiden.

Der vorteilhafte Extremfall einer Banddickenänderung des Fasteningträgers im Schließbereich führt im Sinne der oben vorgeschlagenen Lösung dazu, dass das Band dort teilweise vollständig entfällt, dass also ein Fenster im Fasteningträger angeordnet ist. Ein solches Fenster kann, ebenso wie de vorstehend beschriebene Ausnehmung, insbesondere eine geometrisch einfache Figur aufweisen, wie eine Rechteckform oder eine Kreisform.

Andererseits können auch optisch und haptisch auffälligere Ausnehmungen als Fenster im Fasteningträger angeordnet werden.

In diesem Zusammenhang sei erläutert, dass die Begriffe "oben" und "unten" in Bezug auf die Anordnung der mechanischen Verschlusssystem-Komponente auf dem Fasteningträger dahingehend zu verstehen sind, dass die mechanische Verschlusssystem-Komponente auf dem Fasteningträger, d.h. oberhalb des Fasteningträgers, angeordnet ist. Insoweit bezeichnet der Begriff "unter" dann jeweils eine Baugruppe, die entsprechend unterhalb und mithin eher fasteningträgerseitig einer anderen Baugruppe angeordnet ist, während der Begriff "über" jeweils eine Baugruppe bezeichnet, die entsprechend oberhalb und mithin eher auf einer dem Fasteningträger abgewandten Seite einer anderen Baugruppe angeordnet ist.

Der vorgestellte Aspekt der Erfindung geht einen neuen Weg: Bislang wurden Variierungen im Schließbereich in zahlreichen Varianten an der mechanischen Verschlusssystem-Komponente vorgenommen, so wurden beispielsweise inselförmige Flecken mit Haken oder Schlaufen angeordnet, es wurden bei Hakenflächen die Haken teilweise niedergebügelt, es wurden streifenförmige Hakenflächen aufgeklebt, und vieles andere mehr. Demgegenüber verändert der vorgestellte Aspekt der Erfindung den Fasteningträger, also das Band, welches an der mechanischen Verschlusssystem-Komponente angeordnet ist und diese trägt.

Dies eröffnet völlig neue Wege bei der haptischen und der mechanischen Gestaltung von Verschlussbändern. Unter anderem kann der Fasteningträger auch mit weniger Werkzeugverschleiß bearbeitet werden als der - gegenüber dem Fasteningträger in der Regel härtere - Kunststoff des mechanischen zwei-Komponenten-Verschlusssystems.

Die Erfindung ist nicht auf Hygieneartikel begrenzt. So können entsprechende Verschlussbänder auch in zahlreichen anderen Anwendungen mit einer Komponente eines mechanischen zwei-Komponenten-Verschlusssystems ausgerüstet sein, wobei eine solche Verschlusssystem-Komponente auf einem Stück des Bands angeordnet ist, also auf einem Fasteningträger. Als ein Beispiel für eine alternative Einsatzmöglichkeit eines solchen Verschlussbandes sei unter anderem an einen Kabelbinder gedacht.

Es wird vorgeschlagen, dass die Ausstanzung bzw. das Fenster unter einer Basis der mechanischen Verschlusssystem-Komponente im Schließbereich angeordnet ist. Dementsprechend löst nach einem dritten Aspekt der Erfindung die gestellte Aufgabe auch ein Verschlussband für einen Hygieneartikel mit einem Befestigungsbereich zum permanenten Befestigen am Hygieneartikel und mit einem Schließbereich zum gleichzeitigen lösbaren Verbinden mit einer Oberfläche des Hygieneartikels mit einer auf einem Fasteningträger getragenen Komponente eines mechanischen zwei-Komponenten-Verschlusssystems, welche sich dadurch auszeichnet, dass der Fasteningträger im Schließbereich ein Fenster aufweist, welches unter einer Basis der Verschlusssystem-Komponente angeordnet ist. Eine solche Basis ist in der Regel eine durchgehende Kunststofffläche, aus welcher beispielsweise die Haken oder Schlaufen eines Haken-Schlaufen-Verschlusssystems hervorstehen. Eine solche Basis gibt den Haken eine stabile, aufrechte Ausrichtung und hält alle Haken eines Schließbereichs zusammen. Insbesondere lassen sich die Haken so leichter auf einen Fasteningträger aufkleben.

Wenn die Ausstanzung bzw. das Fenster des Fasteningträgers unter einer solchen Basis angeordnet ist, können die bewährten Verfahren zum Aufbringen der Haken mit einer gemeinsamen Basisfläche auf einem Fasteningträger beibehalten werden. Dennoch kann das Verschlussband gegenüber bisher bekannten Verschlussbändern in seinen mechanischen Eigenschaften verändert sein, weil die Bandfläche des Fasteningträgers unterhalb der Basis nun diskontinuierlich ausgeführt ist. So kann der Fasteningträger unterhalb der Basis der mechanischen Verschlusssystem-Komponente beispielsweise leicht mit Ausstanzungen oder Fenster versehen werden, und das gesamte Verschlussband behält im Schließbereich durch die durchgehende Basis dennoch eine ausreichende Stabilität.

Wie bereits vorstehend erläutert, wird vorgeschlagen, dass die Verschlusssystem-Komponente über der Ausstanzung bzw. über dem Fenster kontinuierlich ist. Es wurde bereits erläutert, dass eine Verschlusssystem-Komponente in einem Schließbereich eines Verschlussbandes oft als einstückiges Element aufgebracht wird. Dies ist ein sehr ökonomisches Verfahren, und es erfordert keine Nachbehandlung mehr, wenn die Verschlusssystem-Komponente darauf kontinuierlich belassen wird.

Bei geeigneter Gestaltung lässt sich für einen Benutzer der Windel die Rückseite der mechanischen Verschlusssystem-Komponente sehr leicht optisch wie haptisch erkennen, was beim Benutzer schon instinktiv dazu führt, dass er zum Anlegen der Windel bzw. konkret zum lösbaren Verbinden des zwei-Komponenten-Verschlusssystems genau auf diese Stelle einen Schließdruck ausübt. Dies lässt die beiden Komponenten des mechanischen Verschlusssystems betriebssicher ineinander gleiten. In der Folge ist sogar davon auszugehen, dass kleinere mechanische Schließbereiche als bisher notwendig verwendet werden können, weil auf die vorhandenen Schließflächen gezielterer Druck vom Benutzer ausgeübt wird.

Um die Rückseite der mechanischen Verschlusssystem-Komponente haptisch bestmöglich erkennbar zu machen, wird vorgeschlagen, dass dort eine Stanzkante im Fasteningträger angeordnet ist. Eine Stanzkante führt zu einer abrupten Änderung der Dicke des Bandmaterials des Fasteningträgers. Konkret kann eine Stanzkante über die gesamte Dicke des Bandes des Fasteningträgers im Schließbereich verlaufen, so dass der Fasteningträger dort abrupt abreißt. Der Benutzer fühlt eine solche Stanzkante recht deutlich und wird umso mehr veranlasst, das Verschlussband genau dort zu ergreifen und mit Druck an dieser Stelle die Windel zu verschließen.

Um die Rückseite der mechanischen Verschlusssystem-Komponente unabhängig von der Haptik optisch gut erkennbar zu machen, wird vorgeschlagen, dass der Fasteningträger eine andere Farbe als die Verschlusssystem-Komponente aufweist. Sowohl der Bandwerkstoff des Fasteningträgers als auch der Kunststoff der mechanischen Verschlusssystem-Komponente kann in praktisch jeder Farbe hergestellt werden. In der Regel werden möglichst ähnliche Farben verwendet, um das Verschlussband optisch nicht zu unruhig werden zu lassen. Demgegenüber wird nun vorgeschlagen, zwischen dem Fasteningträger - insbesondere der von der Verschlusssystem-Komponente abgewandten Seite des Fasteningträgers - und der mechanischen Verschlusssystem-Komponente - insbesondere ihrer zum Fasteningträger gewandten Unterseite - einen Farbunterschied vorzusehen, bevorzugt einen möglichst großen Farbkontrast. So kann beispielsweise der Fasteningträger aus einem eher weißen Band bestehen und die mechanische Verschlusssystem-Komponente einen dunkelblauen Kunststoff aufweisen.

Bei geeigneter Gestaltung führt die Ausnehmung des Fasteningträgers im Schließbereich dazu, dass die Farbe der Verschlusssystem-Komponente zu der ihr abgewandten Seite des Fasteningträgers dort durchscheint, wo der Fasteningträger beispielsweise verdünnt oder im Idealfall vollständig ausgestanzt ist. Auf derjenigen Seite des Fasteningträgers, welche von der Verschlusssystem-Komponente abgewandt ist, wäre bei der beispielhaft genannten Farbgebung auf dem ansonsten weißen Band des Fasteningträgers genau auf der Rückseite der Hakenfläche ein blauer Fleck ersichtlich, beispielsweise ein blauer Punkt oder ein blauer Stern. Es ist unmittelbar vorstellbar, dass ein Benutzer beim Anlegen der Windel genau an dieser Stelle den Druck zum Verschließen ausüben wird.

Sowohl optisch als auch haptisch kann der Ort des Schließbereichs auf der Rückseite des Fasteningträgers auch dadurch erkennbar gemacht werden, dass der Fasteningträger dort eine Werkstoffänderung aufweist. So ist beispielsweise vorstellbar, dass der Fasteningträger als ein durchlaufendes Band hergestellt ist, bei welchem an ein eher dunkles, nicht optisch durchlässiges Bandmaterial ein farbloses, optisch durchlässiges Stück a-nextrudiert ist, und dass das Band nach einem kurzen Abschnitt des farblosen Bandes wieder mit Anextrusion eines dunklen Stücks fortgeführt wird. Auch können gezielt verschieden Fasern zur Anwendung kommen, wenn der Fasteningträger aus Fasern gebildet ist bzw. solche umfasst. Auf diese Weise wäre der Fasteningträger zwar in der Fläche kontinuierlich, würde aber optisch unter einem Teilbereich eine dort liegende Farbe erkennbar machen. So würde eine solche Gestaltung bei der vorstehend beschriebenen Farbenkombination dazu führen, dass der Fasteningträger unterhalb des Schließbereichs auf der der mechanischen Verschlusssystem-Komponente abgewandten Seite blau durchschimmert.

Es wurde bereits erläutert, dass zwischen dem Fasteningträger und der Verschlusssystem-Komponente oft eine Klebstoffschicht angeordnet ist, um die beiden Bauelemente aneinander sicher zu fixieren. In diesem Fall wird vorgeschlagen, dass die Klebstoffschicht zwischen dem Fasteningträger und der Verschlusssystem-Komponente eine Mitausnehmung aufweist. Diese kann von derselben Qualität sein wie die Ausstanzung bzw. das Fenster des Fasteningträgers, also eine bevorzugt deckungsgleiche vollständige Aussparung. Hier führt beispielsweise letzteres dazu, dass nicht über einem Fenster im Bandmaterial des Fasteningträgers Klebstoff frei an der Oberfläche liegt, was schon deshalb negativ wäre, weil sich dort leicht Verschmutzungspartikel versetzen könnten. Diese sind beim Ausziehen einer Windel regelmäßig zu erwarten.

Es versteht sich, dass von den vorgestellten Aspekten und möglichen Variationen der Erfindung ein Bandmaterial unmittelbar profitiert, welches eine Längserstreckung aufweist und senkrecht zur Längserstreckungsrichtung und bevorzugt auch in Längserstreckungsrichtung zu Einzelnutzen durchtrennt werden kann, bevorzugt ohne Verschnitt. Ein solches Bandmaterial ist die übliche Lieferform für Windelverschlussbänder, wie sie bei einem Windelhersteller verarbeitet werden. Das Bandmaterial durchläuft in seiner Längserstreckungsrichtung die Ansetzmaschine in Maschinenlaufrichtung. Messer schneiden das Bandmaterial in senkrechter Richtung hierzu durch, also in sogenannter cross-direction.

Es wurde bereits erwähnt, dass ein vorteilhaftes Bandmaterial als Wicklung vom Bandhersteller zum Windelhersteller transportiert werden kann, insbesondere als Rolle oder Spule.

Es versteht sich, dass die Vorteile des Bandmaterials unmittelbar auf eine Wicklung und selbstverständlich ebenso auf eine Windel mit einem Verschlussband durchschlagen.

Nach einem weiteren Aspekt der Erfindung löst die Aufgabe ein Verfahren zum Herstellen eines Verschlussbandes für einen Hygieneartikel oder zum Herstellen eines Bandmaterials, wobei aus einem Fasteningträger ein Stück ausgestanzt wird, bevorzugt gemeinsam mit darauf angeordnetem Klebstoff. Ein solches Herstellungsverfahren ist sehr wirtschaftlich. So kann der Fasteningträger vollflächig mit Klebstoff beschichtet werden, und aus dem beschichteten Träger kann dann gezielt ein Stück ausgestanzt werden. Auf diese Weise ist es auch möglich, verschiedene Motive allein durch Wechseln des Stanzwerkzeugs auszustanzen, ohne eine Veränderung in der vorherigen Herstellung des kontinuierlichen Fasteningträgerbandes oder der Klebstoffbeschichtung vornehmen zu müssen.

Die Erfindung wird nachfolgend anhand eines Ausführungsbeispiels unter Bezugnahme auf die Zeichnung näher erläutert. Es zeigen
- Fig. 1: schematisch eine Seitenansicht auf ein Windelverschlussband mit einem ausgestanzten Fasteningträger, wobei das Windel- verschlussband an einer Windel befestigt ist,
- Fig. 2: ein Benutzerende des Windelverschlussbands aus Fig. 1 ge- mäß dortiger Kennzeichnung II-II und
- Fig. 3: das Windelverschlussband aus Fig. 1 in einer vergleichbaren Draufsicht wie in Fig. 2, jedoch in einer anderen Ausführungs- form.

Das Windelverschlussband 1 in den Figuren besteht im Wesentlichen aus einem inelastischen ersten Bandabschnitt 2, einem elastischen Bandabschnitt 3 und einem inelastischen Fasteningträger 4 mit einem daran angebrachten Hakenabschnitt 5.

Das inelastische Band 2 ist auf seiner zu einem Windelohr 6 gerichteten Seite vollständig mit einer Klebstoffschicht 7 beschichtet und über die Klebstoffschicht 7 an einer Oberfläche 8 des Windelohrs 6 permanent angeschlossen. Der größte Teil 9 des Bandes 2 dient somit zum permanenten Befestigen des Windelverschlussbands 1 am Windelohr 6.

An einem überstehenden Ende 10 des ersten Bandes 2 ist die Klebstoffschicht 7 an dem elastischen Bandabschnitt 3 angeklebt. Statt einer Klebeverbindung können je nach Ausführungsform hier auch andere Verbindungsarten zur Anwendung kommen. Er gibt dem Windelverschlussband 1 insgesamt in einer Längserstreckungsrichtung 11 ein elastisches Verhalten.

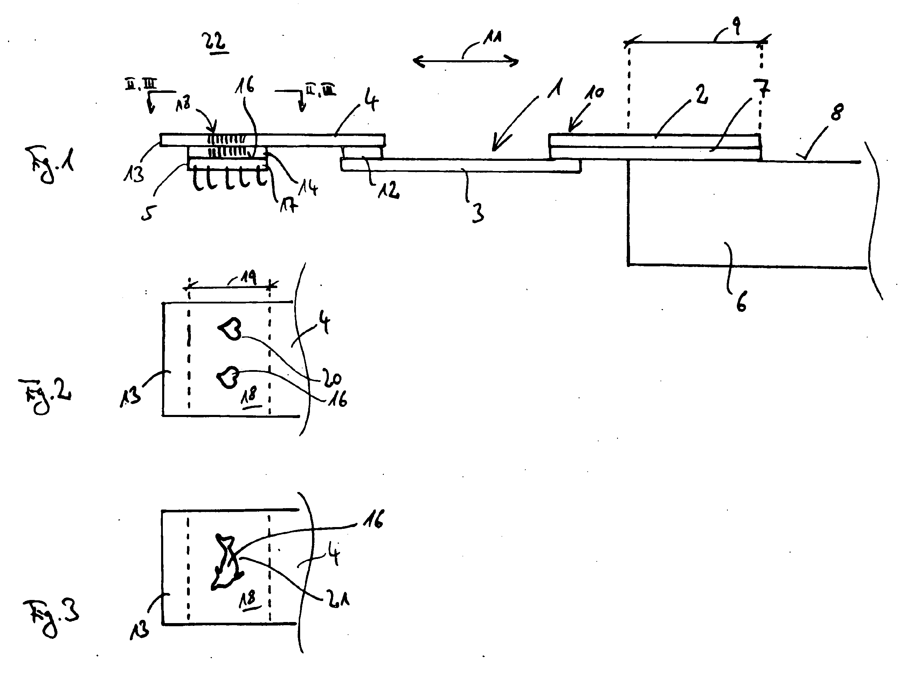

Der elastische Bandabschnitt 3 ist mit einem Klebstoffstreifen 12 seinerseits an den Fasteningträger 4 angeklebt, wobei auch hier andere Verbindungsarten zur Anwendung kommen können. Vor einem freien Überstandsabschnitt 13 ist eine weitere Klebefläche 14 am Fasteningträger 4 angeordnet. Die Klebefläche 14 (in den Fig. 2 und 3 an der verdeckten Seite des Bandes 1) fixiert über Kraftschluss mit einer Bodenfläche 16 eine Basis 17 des Hakenbereichs 5 am Fasteningträger.

Es versteht sich, dass vorliegende Erfindung auch unabhängig vom Vorhandensein eines elastischen Bandabschnittes 3 vorteilhaft ist und insbesondere auch bei inelastischen Windelverschlussbändern zur Anwendung kommen kann.

Insgesamt kann das Verschlussband 1 dazu benutzt werden, eine Windel betriebssicher lösbar zu verschließen. Hierzu wird die Windel dem Träger angelegt, und anschließend werden die Haken des Hakenbereichs 5 mit einer korrespondierenden Schlaufenschar an einem Frontaltape der Windel in Eingriff gebracht. Dies geschieht durch ein Auflegen der Haken des Hakenbereichs 5 auf den Schlaufenbereich (nicht dargestellt) und ein anschließendes Ausüben von Druck auf eine hakenabgewandte Seite 18 im Bereich unter den Haken 5, also in einem Schließbereich 19.

Im Schließbereich 19 ist durch das Band des Fasteningträgers 4 und den darüber angeordneten Kleber 14 eine Ausstanzung in beliebiger Form angeordnet (in Fig. 1 durch parallele Striche gekennzeichnet), so beispielsweise in Form mehrerer Herzen 20 (vergleiche die Ausführungsform in Fig. 2) oder in Form eines Delphins 21 (vergleiche die alternative Ausführungsform in Fig. 3).

Der Fasteningträger 4 ist auf seiner Oberseite 18 sehr hell, nahezu weiß, in der Farbe. Er ist im Wesentlichen lichtundurchlässig. Die Unterseite 16 der Basis 17 des Hakenmaterials 5 ist demgegenüber aus dunkelblauem Kunststoff und ebenfalls weitgehend lichtundurchlässig. Daher lässt sich von einer Bedieneraußenseite 22 bei Blick auf das Benutzerende 3,4 des Windelverschlussbands 1 die jeweilige Stanzform 20, 21 als deutlich blau ausgefüllt erkennen, weil durch die Stanzung 20,21 ein Fenster direkt auf die Unterseite 16 der Hakenbasis 17 freigegeben ist. Im Übrigen Bereich 18 des Fasteningträgers 4 im Schließbereich 19 erscheint die eigentliche Oberfläche des Fasteningträgers, so dass somit dieser Bereich 18 im Übrigen weiß ist.

Der Benutzer sieht hierdurch nicht nur von seiner Außenseite 22 exakt, wo er beim Anlegen der Windel Druck ausüben muss, um die Haken fest in korrespondierende Schlaufen zu drücken. Vielmehr spürt er auch die scharfen Stanzkanten 20,21 mit seinen Fingern und wird dazu tendieren, die Daumen an dieser Position zu belassen, während ein Zeigefinger beispielsweise an den Überstandsabschnitt 13 angelegt wird.

In der Regel wird die Ausstanzung 20, 21 in der Klebstoffschicht 14 deckungsgleich mit der Ausstanzung im Fasteningträger 4 sein, weil in der Regel der Fasteningträger 4 mit Klebstoff 14 beschichtet wird, und nicht der Klettstreifen 5. Auf Wunsch kann dies jedoch auch geändert werden, so dass die Klebefläche 14 kontinuierlich bliebe, während in den Fasteningträger 4 ein Fenster gestanzt würde. Dies hätte zur Folge, dass durch die Fenster 20, 21 zur Außenseite 22 hin die Klebefläche 14 offen läge. Dies könnte zu einem besseren und gründlicheren Griff am Benutzerende des Windelverschlussbands 1 führen, insbesondere auch bei widrigen Schmutzbedingungen am Verschlussband 1.

Beim Herstellungsverfahren können die herausgestanzten Teile des Fasteningträgers - ob mit oder ohne Klebstoffschicht 14 - bevorzugt durch Absaugen, Abgittern oder mit Hilfsmitteln wie Linern abgenommen werden.

Insgesamt lässt sich allerdings ein Windelverschlussband 1 mit einer solchen Ausstanzung sehr kostengünstig herstellen und führt zu überraschenden Verbesserungen der Benutzbarkeit einer Windel.

## Patentansprüche

1. Verschlussband (1) mit einer auf einem Fasteningträger (4) getragenen Komponente (5) eines mechanischen zwei-Komponenten-Verschlusssystems, ***dadurch gekennzeichnet, dass*** der Fasteningträger (4) unter der Verschlusssystem-Komponente (5) eine Ausstanzung (20, 21) aufweist.

2. Verschlussband nach Anspruch 1, ***dadurch gekennzeichnet, dass*** die Ausstanzung (20, 21) unter einer Basis (17) der Verschlusssystem-Komponente (5) angeordnet ist.

3. Verschlussband nach Anspruch 1 oder 2, ***dadurch gekennzeichnet, dass*** die Verschlusssystem-Komponente (5) über der Ausstanzung (20, 21) kontinuierlich ist.

4. Verschlussband nach einem der vorhergehenden Ansprüche, ***gekennzeichnet durch*** eine Stanzkante im Fasteningträger (4) im Schließbereich (19).

5. Verschlussband nach einem der vorhergehenden Ansprüche, ***dadurch gekennzeichnet, dass*** der Fasteningträger (4) eine andere Farbe als die Verschlusssystem-Komponente (16) aufweist.

6. Verschlussband nach einem der vorhergehenden Ansprüche, ***dadurch gekennzeichnet, dass*** eine Klebstoffschicht (14) zwischen dem Fasteningträger (4) und der Verschlusssystem-Komponente (5) eine Mitausnehmung (20, 21) aufweist.

7. Bandmaterial mit einer Längserstreckungsrichtung, welches durch Trennen zu Einzelnutzen in Form von Verschlussbändern (1) nach einem der vorhergehenden Ansprüche aufteilbar ist, indem das Bandmaterial quer (11) zur Längserstreckungsrichtung und gegebenenfalls auch im wesentlichen in Längserstreckungsrichtung durchtrennt werden kann.

8. Wicklung von Bandmaterial nach Anspruch 7.

9. Wicklung nach Anspruch 8 in Form einer Rolle.

10. Wicklung nach Anspruch 8 in Form einer Spule.

11. Windel mit einem Verschlussband nach einem der Ansprüche 1 bis 6.

12. Verfahren zum Herstellen eines Verschlussbandes (1) nach einem der Ansprüche 1 bis 6 oder eines Bandmaterials nach Anspruch 7, ***dadurch gekennzeichnet, dass*** aus einem Fasteningträger (4) ein Stück ausgestanzt wird.

13. Verfahren nach Anspruch 12, ***dadurch gekennzeichnet, dass*** aus einem Fasteningträger (4) gemeinsam mit darauf angeordnetem Klebstoff (14) ein Stück ausgestanzt wird.

## Claims

1. A sealing strip (1) with a component (5) of a mechanical two-component sealing system, carried on a fastening support (4), **characterised in that**, the fastening support (4) has a punched-out shape (20, 21) under the sealing system component (5).

2. The sealing strip in accordance with Claim 1, **characterised in that**, the punched-out shape (20, 21) is arranged under a base (17) of the sealing system component (5).

3. The sealing strip in accordance with Claim 1 or 2, **characterised in that**, the sealing system component (5) is continuous above the punched-out shape (20, 21).

4. The sealing strip in accordance with one of the preceding claims, **characterised by** a punched edge in the fastening support (4) in the sealing region (19).

5. The sealing strip in accordance with one of the preceding claims, **characterised in that**, the fastening support (4) has a colour other than that of the sealing system component (16).

6. The sealing strip in accordance with one of the preceding claims, **characterised in that**, an adhesive layer (14) between the fastening support (4) and the sealing system component (5) has an accompanying opening (20, 21).

7. A strip material with a direction of longitudinal extent, which by means of separation for purposes of individual usage can be divided in the form of sealing strips (1) in accordance with one of the preceding claims, in that the strip material can be severed transverse (11) to the direction of longitudinal extent and, if required also essentially in the direction of longitudinal extent.

8. A winding of strip material in accordance with Claim 7.

9. A winding in accordance with Claim 8 in the form of a roll.

10. A winding in accordance with Claim 8 in the form of a spool.

11. A nappy with a sealing strip in accordance with one of the Claims 1 to 6.

12. A method for the manufacture of a sealing strip (1) in accordance with one of the Claims 1 to 6, or of a strip material in accordance with Claim 7, **characterised in that**, a piece is punched out of a fastening support (4).

13. The method in accordance with Claim 12, **characterised in that**, a piece, together with an adhesive (14) arranged thereon, is punched out of a fastening support (4).

## Revendications

1. Bande de fermeture (1) comportant un composant (5) supporté par un support de fixation (4) d'un système de fermeture mécanique à bi-composant, **caractérisée en ce que** le support de fixation (4) présente une découpe (20, 21) sous le composant du système de fermeture (5).

2. Bande de fermeture selon la revendication 1, **caractérisée en ce que** la découpe (20, 21) est disposée sous une base (17) du composant du système de fermeture (5).

3. Bande de fermeture selon la revendication 1 ou 2, **caractérisée en ce que** le composant du système de fermeture (5) est continu au-dessus de la découpe (20, 21).

4. Bande de fermeture selon une des revendications précédentes, **caractérisée par** une arête de coupe (4) se situant dans le support de fixation au niveau de la fermeture (19).

5. Bande de fermeture selon une des revendications précédentes, **caractérisée en ce que** le support de fixation (4) a une autre couleur que le composant du système de fermeture (16).

6. Bande de fermeture selon une des revendications précédentes, **caractérisée en ce qu'**une couche de colle (14) présente un évidement connexe (20, 21) entre le support de fixation (4) et le composant du système de fermeture (5).

7. Matériau en bande ayant un sens d'extension longitudinal, qui peut être divisé par sectionnement pour usage unique sous la forme de bandes de fermeture (1) selon une des revendications précédentes par le fait que le matériau en bande peut être sectionné transversalement (11) par rapport au sens d'extension longitudinal et le cas échéant aussi sensiblement dans le sens d'extension longitudinal.

8. Bobinage de matériau en bande selon la revendication 7.

9. Bobinage selon la revendication 8 sous la forme d'un rouleau.

10. Bobinage selon la revendication 8 sous la forme d'une bobine.

11. Couche comportant une bande de fermeture selon une des revendications 1 à 6.

12. Procédé de fabrication d'une bande de fermeture (1) selon une des revendications 1 à 6 ou d'une bande de matériau selon la revendication 7, **caractérisé en ce qu'**un morceau est découpé dans un support de fixation (4).

13. Procédé selon la revendication 12, **caractérisée en ce qu'**un morceau avec de la colle (14) disposée dessus est découpé dans un support de fixation (4).
